(19) Europäisches Patentamt
European Patent Office
Office européen des brevets

(11) **EP 4 387 467 B1**

(12) **EUROPEAN PATENT SPECIFICATION**

(45) Date of publication and mention
of the grant of the patent:
**11.03.2026 Bulletin 2026/11**

(21) Application number: **22769108.6**

(22) Date of filing: **22.08.2022**

(51) International Patent Classification (IPC):
*A23L 2/60* (2006.01)    *A23L 27/30* (2016.01)
*A23L 27/00* (2016.01)    *A23L 33/185* (2016.01)
*C07K 14/43* (2006.01)

(52) Cooperative Patent Classification (CPC):
**A23L 2/60; A23L 27/31; A23L 27/88; C07K 14/43**

(86) International application number:
**PCT/EP2022/073331**

(87) International publication number:
**WO 2023/021218 (23.02.2023 Gazette 2023/08)**

(54) **SWEETENER BLEND COMPRISING THAUMATIN AND BRAZZEIN**

SÜSSSTOFFMISCHUNG MIT THAUMATIN UND BRAZZEIN

MÉLANGE ÉDULCORANT COMPRENANT DE LA THAUMATINE ET DE LA BRAZZÉINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2021 US 202163235262 P**

(43) Date of publication of application:
**26.06.2024 Bulletin 2024/26**

(73) Proprietor: **Nomad Bioscience GmbH
80333 München (DE)**

(72) Inventors:
• **STEPHAN, Anett
06118 Halle (Saale) (DE)**
• **GIRITCH, Anatoli
06108 Halle (Saale) (DE)**
• **GLEBA, Yuri
10117 Berlin (DE)**
• **HAHN-LÖBMANN, Simone
06217 Merseburg (DE)**
• **PROCHASKA, Heike
06114 Halle (Saale) (DE)**

(74) Representative: **Wächtershäuser & Hartz
Patentanwaltspartnerschaft mbB
Weinstraße 8
80333 München (DE)**

(56) References cited:
WO-A1-2022/012926    US-A1- 2013 183 427
US-A1- 2013 183 427    US-A1- 2020 107 568
US-A1- 2020 107 568

Remarks:
The complete document including Reference Table(s) and the Sequence Listing(s) can be downloaded from the EPO website

EP 4 387 467 B1

**Description**

**FIELD OF THE INVENTION**

The present invention relates to a low-calorie sweetener composition having sweetness synergy and improved organoleptic properties comprising the sweet proteins Thaumatin, such as Thaumatin II, and Brazzein. The present invention also relates to food and beverage products comprising said sweetener composition and a method of manufacturing a reduced-calorie product.

**BACKGROUND**

Most food and beverage products contain nutritive sweeteners such as sucrose (generally referred to as 'sugar' or 'table sugar'), glucose, fructose, corn syrup, high fructose corn syrup and the like, or high intensity sweeteners such as aspartame, sucralose, acesulfame K, saccharin, cyclamates, steviol glycosides and the like. Such sweeteners supply sweetness and a favorable sensory response, for example in terms of flavor balance, quality of sweetness, lack of bitterness and off taste, desirable temporal profile and desirable mouthfeel.

Despite the preferred taste and functional properties provided by nutritive sweeteners like sugar, excess intake of full calorie sweeteners has long been associated with an increase in diet-related health issues, such as obesity, heart disease, metabolic disorders and dental problems. This worrying trend has caused consumers to become increasingly aware of the importance of adopting a healthier lifestyle and reducing the level of nutritive sweeteners in their diet.

Recently there has been a substantial increase in consumer products utilizing replacements for nutritive sweeteners, with a particular focus on the development of low or zero-calorie sweeteners of natural origin. In fact, in North America, the number of new product launches utilizing natural high intensity low calorie sweeteners has increased, outpacing artificially sweetened new product launches, while nutritive sweetened product launches have slowly declined.

An ideal replacement for a nutritive or artificial sweetener is a sweetener that has desirable taste characteristics with fewer calories, can be sustainably produced, has clearly understood metabolism and history of safe use, and is cost effective. Aiming to meet this growing need, the market has been flooded with possible candidates to replace conventional nutritive sweeteners. Unfortunately, however, many of the low or zero calorie replacements offered on the market lack one or all of the necessary characteristics, and often exhibit bitterness or off-taste. Therefore, many of the proposed sweeteners are not an ideal replacement for nutritive sweeteners.

Thaumatin, one of a very few known sweet proteins, is found in Katemfe fruit and is well known as a sweetener and flavor modifier. However, as Katemfe fruit is not readily cultivated, sustainability of the thaumatin market is poor and cost is high. Additionally, thaumatin from Katemfe fruit is a mixture of thaumatin proteins, some of which have better organoleptic properties such as thaumatin II, and some which do not have preferred organoleptic properties. This is further complicated as thaumatin from katemfe fruit is a natural product and the ratios of thaumatins are variable in addition to the quality. Furthermore, while thaumatin alone has good taste properties when providing a sweetness equivalent to a 5% solution of sucrose, at higher sweetening concentrations it suffers from off taste properties such as bitter, metallic, or medicinal notes. Recent advances in technology allow the production of thaumatin through transient expression of this protein in well-known agricultural commodity crops which has eliminated the variability, mixed nature, and reduced the cost in use resulting in a readily available source of thaumatin II, which has improved organoleptic properties vs. mixed thaumatins. However, at higher sweetening concentrations, thaumatin II from commodity crop expression still suffers from off taste properties, thus necessitating combination with other sweeteners.

Brazzein, like thaumatin is also one of the very few known sweet proteins and also comes from a fruit. Brazzein comes from the Oubli fruit (*Pentadiplandra brazzeana Baillon*) found in West Africa. Brazzein has yet to have been successfully commercialized in a meaningful way despite its excellent sweetness characteristics primarily due to its long sweet linger, and difficulty in cultivation of the Oubli fruit. The latter limitation can be alleviated by heterologous production in a microorganism via fermentation. Brazzein has the added benefit of being extremely heat stable. However, the long sweet linger and delayed temporal profile, makes Brazzein on its own not suitable as a standalone sweetener.

Therefore, there is a no standalone sweetening solution comprised of protein sweeteners that meets the organoleptic needs for products at high sweetness levels. There is still a need to provide an improved replacement of natural origin for nutritive sweeteners that has low or zero-calories and is without limitations in use, but which also has preferred taste characteristics, is sustainable, has high solubility, and good cost in use.

US 2013/183427 Al relates to a composition for sweetening an edible product, comprising thaumatin, characterized in that it further comprises gymnemic acid. US 2020/107568 A1 describes a method for reducing a sweet lingering produced by a high sweetness sweetener and a composition to be used in the method. Thaumatin and brazzein are mentioned therein.

## SUMMARY OF THE INVENTION

The present invention seeks to provide a solution to the above-mentioned problems by providing a sweetener or flavor modifying composition comprising protein sweeteners of natural origin, acceptable cost in use, preferred organoleptic properties, history of safe use, and low-calorie content. The present invention also seeks to provide a sweetener composition having sweetness synergy, a reduction in off tastes or off flavors, desirable temporal profile, when compared with other high intensity sweeteners of natural origin.

The invention is defined according to the appended claims 1 to 11.

Generally, the present invention provides a sweetener or flavor modifying composition according to claim 1. The sweetener or flavor modifying composition comprises a Thaumatin and a Brazzein, said composition comprising Thaumatin in an amount of about 5% to about 20% and Brazzein in an amount of about 80% to about 95% by weight relative to the combined weight of the Brazzein and Thaumatin portion of the composition. The Thaumatin may be selected from Thaumatin I, Thaumatin II, Thaumatin A, and Thaumatin B. The Brazzein may be selected from type 1 Brazzein, type 2 Brazzein and type 3 Brazzein. In one embodiment, the Thaumatin is Thaumatin I or Thaumatin II, and the Brazzein is selected from type 1 Brazzein and type 3 Brazzein, preferably the Thaumatin is Thaumatin II and the Brazzeine is type 2 Brazzein.

The thaumatin II may be of a purity greater than 90% and Brazzein may be of a purity greater than 90%. The sweetener or flavor modifying composition may comprise Thaumatin II and Brazzein.

The sweetener or flavor modifying composition may comprise Thaumatin, preferably Thaumatin II, of a purity greater than 90% in an amount of about 5% to about 20%, and Brazzein of a purity greater than 90% in an amount of about 80% to about 95% by weight relative to the total weight of the combined Thaumatin and Brazzein portion of the composition.

The sweetener or flavor modifying composition comprises Thaumatin, preferably Thaumatin II in an amount of about 5% to about 20%, and Brazzein in an amount of about 80% to about 95% by weight relative to the total weight of the combined Thaumatin and Brazzein portion of the composition.

The sweetener or flavor modifying composition may comprise Thaumatin, preferably Thaumatin II, of a purity greater than 90% in an amount of about 15% and Brazzein in an amount of about 85% by weight relative to the combined weight of the Thaumatin and Brazzein portion of the sweetening or flavor modifying composition.

In further embodiment, the sweetener or flavor modifying composition comprises Thaumatin, preferably Thaumatin II, in an amount of about 15% and Brazzein in an amount of about 85% by weight relative to the combined weight of the Thaumatin and Brazzein portion of the sweetening or flavor modifying composition.

The sweetener or flavor modifying composition may comprise Thaumatin, preferably Thaumatin II, in an amount of about 10% and Brazzein in an amount of about 90% by weight relative to the combined weight of the Brazzein and Thaumatin portion of the sweetening or flavor modifying composition.

The thaumatin II portion utilized in the composition may be greater than 90% purity relative to the weight of the total thaumatin II portion of the composition. Additionally, the Brazzein portion utilized in the composition may be greater than 90% purity relative to the weight of the total Brazzein portion of the composition.

The sweetener or flavor modifying composition may further comprise another sweet tasting additive, a stabilizing or solubilizing solvent, a bulking agent, a flavoring agent, and/ or a stabilizer.

The food or beverage product comprises a sweetener or flavor modifying composition of the invention.

The sweetener or flavor modifying composition may be used in the resulting food or beverage product comprises Thaumatin, preferably Thaumatin II, at a concentration in the food or beverage product at about 0.2 ppm to about 10 ppm and Brazzein at a concentration in the food or beverage product at about 1 ppm to about 200 ppm in the food or beverage product. The total concentration of the combined thaumatin and brazzein sweetener or flavor modifying composition is about 1.2 ppm to about 210 ppm in the resulting food or beverage product.

Further aspects of the present invention provide a cosmetic product comprising a sweetener composition according to the invention; a pharmaceutical product comprising a sweetener composition according to the invention; and a nutritional product comprising a sweetener composition according to the invention[0021] The sweetener or flavor modifying composition according to the present invention may be used in a food product, a beverage product, a pharmaceutical product, a nutritional product, a sports product, or a cosmetic product.

## BRIEF DESCRIPTION OF THE FIGURES

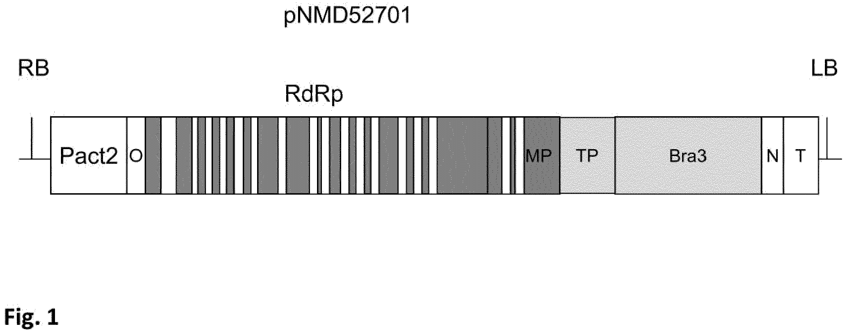

Figure 1 shows schematically the T-DNA region of pNMD52701 construct for the expression of Brazzein type 3. The expression vector is based on Tobacco mosaic virus (TMV). RB and LB stand for the right and left borders of T-DNA of binary vector. Pact2: promoter of Arabidopsis actin2 gene; o: 5' end from TVCV (turnip vein clearing virus); RdRp:

RNA-dependent RNA polymerase open reading frame (ORF) from cr-TMV (crucifer-infecting tobamovirus); MP: movement protein ORF from cr-TMV; TP: apoplast targeting presequence from rice alpha-amylase 3A; Bra3: coding sequence of mature Brazzein type 3; N: 3'-non-translated region from cr-TMV; T: Agrobacterium nopaline synthase terminator; white segments interrupting grey segments in the RdRp and MP ORFs indicate introns inserted into these ORFs for increasing the likelihood of RNA replicon formation in the cytoplasm of plant cells, which is described in detail in WO2005049839.

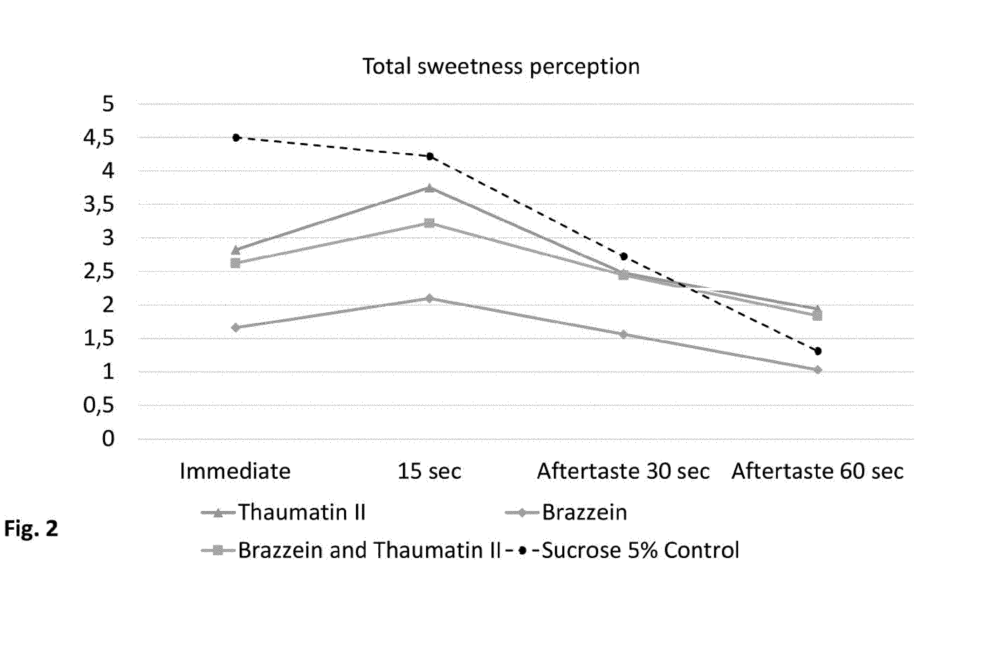

**Figure 2** illustrates the total mean sweet perception for Thaumatin II, Brazzein, and the combination thereof. 5% sucrose solution in water was used as a sweetness control.

## DETAILED DESCRIPTION

The present invention is based upon the discovery that Thaumatin, notably Thaumatin II, surprisingly has unique characteristics when highly purified from other thaumatins and combined with Brazzein. Thaumatin exists naturally as several isoforms in Katemfe fruit (Thaumatococcus daniellii). Thus, the term Thaumatin is generic and relates to any isoform or mixture of two or more isoforms of Thaumatin. These isoforms have different levels of sweetness, sweet linger and off taste. Thaumatin II is the isoform which has the best quality of sweetness with the fewest negative organoleptic properties. Therefore, Thaumatin II is the preferred Thaumatin in the present invention. However, it is difficult to separate or enrich Thaumatin II from the natural Katemfe fruit extract mixture of thaumatin isoforms. However, in the present invention, Thaumatin II with, preferably, a purity of greater than 90% by weight has been utilized as isolated by transient recombinant expression in sustainable agricultural commodity crops. This has the dual benefit of only producing the best isoform Thaumatin II and doing so in a sustainable manner. Transient recombinant expression of Thaumatins, including Thaumatin II, is known in the art and is, for example, described in WO 2022/012926 A1.

Surprisingly, when Thaumatin, notably Thaumatin II, preferably of purity greater than 90%, is combined with Brazzein, said composition comprising Thaumatin in an amount of about 5% to about 20% and Brazzein in an amount of about 80% to about 95% by weight relative to the combined weight of the Brazzein and Thaumatin portion of the composition, the composition has an improved quality of taste and a higher relative sweetness or sweetness synergy while being entirely sweetened by protein. This is the first such example of a sweetening mixture consisting entirely of protein, with acceptable organoleptic properties and cost in use. Additionally, the relative sweetness of the sweetener composition is greater than the sweetness calculated from the individual components of the composition and furthermore the negative taste attributes of the individual components are reduced in the final composition, in particular, with regard to the bitter taste and/or undesirable temporal profile that may be associated with the individual components. In addition, due to each of the components contributing near zero calories in use, the sweetener composition is zero or low calorie. Furthermore, as a consequence of the sweetness synergy exhibited by the composition, the amount of the composition required to provide a given level of sweetness is less than would be expected in the absence of synergy, thereby allowing a further reduction in both cost and calories. Thus, the sweetener composition of the present invention provides enhanced sweetness, improves the balance of flavor by reducing off-taste, and provides a more desirable temporal profile, while at the same time allowing a significant reduction in calories and cost compared to a sweet-equivalent amount of a conventional nutritive sweetener or other natural high intensity sweeteners used individually.

Using the sweetener or flavor modifying composition of the present invention allows delivery of an increased sweetness in food or beverage products when compared to the individual components used separately. This enhanced sweetness means that a smaller amount of sweetener can be used in these products, and therefore the cost in use is reduced.

In general terms, the present invention relates to a sweetener or flavor modifying composition comprising Thaumatin, preferably Thaumatin II, and Brazzein. Herein, the generic term "Thaumatin" refers to any one or more of Thaumatin isoforms I, II, A, and B. The term "Thaumatin I" refers to an oligopeptide or protein of the following amino acid sequence **(SEQ ID NO: 1):**

ATFEIVNRCSYTVWAAASKGDAALDAGGRQLNSGESWTINVEPGTNGGKIWARTDCYFDDSGSGICKTGDCGGLLRCKRFGR

PPTTLAEFSLNQYGKDYIDISNIKGFNVPMNFSPTTRGCRGVRCAADIVGQCPAKLKAPGGGCNDACTVFQTSEYCCTTGKCGP

TEYSRFFKRLCPDAFSYVLDKPTTVTCPGSSNYRVTFCPTA

The term "Thaumatin II" refers to an oligopeptide or protein of the following amino acid sequence **(SEQ ID NO: 2):**

ATFEIVNRCSYTVWAAASKGDAALDAGGRQLNSGESWTINVEPGTKGGKIWARTDCYFDDSGRGICRTGDCGGLLQCKRFGR

PPTTLAEFSLNQYGKDYIDISNIKGFNVPMDFSPTTRGCRGVRCAADIVGQCPAKLKAPGGGCNDACTVFQTSEYCCTTGKCGP

TEYSRFFKRLCPDAFSYVLDKPTTVTCPGSSNYRVTFCPTA

The term "Thaumatin A" refers to an oligopeptide or protein of the following amino acid sequence (**SEQ ID NO: 3**):

ATFEIVNRCSYTVWAAASKGDAALDAGGRQLNSGESWTINVEPGTNGGKIWARTDCYFDDSGSGICKTGDCGGLLRCKRFGR

PPTTLAEFSLNQYGKDYIDISNIKGFNVPMDFSPTTRGCRGVRCAADIVGQCPAKLKAPGGGCNDACTVFQTSEYCCTTGKCGP

TEYSRFFKRLCPDAFSYVLDKPTTVTCPGSSNYRVTFCPTA

The term "Thaumatin B" refers to an oligopeptide or protein of the following amino acid sequence **(SEQ ID NO: 4):**

ATFEIVNRCSYTVWAAASKGDAALDAGGRQLNSGESWTINVEPGTKGGKIWARTDCYFDDSGSGICKTGDCGGLLRCKRFGR

PPTTLAEFSLNQYGKDYIDISNIKGFNVPMDFSPTTRGCRGVRCAADIVGQCPAKLKAPGGGCNDACTVFQTSEYCCTTGKCGP

TEYSRFFKRLCPDAFSYVLDKPTTVTCPGSSNYRVTFCPTA

The Thaumatins may be used singly or two or more may be used jointly in the invention. For example, Thaumatin A and Thaumatin B may be used in combination. Alternatively, Thaumatin I and Thaumatin II may be used in combination. Where mixtures of two or more Thaumatins are used, any amounts or ratios with Brazzein refer to the combination of the two or more Thaumatins, unless stated otherwise. However, as Thaumatin II has the most preferred organoleptic properties as a sweetener, Thaumatin II is preferred and the use of Thaumatin II as the sole Thaumatin is most preferred.

The term "Brazzein" is generic and refers to any one, two, or three of type 1, type 2 and type 3 Brazzein. Brazzein is a heat-stable sweet protein from the west African plant *Pentadiplandra brazzeana.* Brazzein is a 6.5-kDa, single-chain polypeptide with four intramolecular disulfide bridges. Three forms of Brazzein are found in the natural source. They differ only at the N-terminal amino acid residue. Type 2 Brazzein corresponds to the predicted 54-amino acid translation product containing a glutamine (Q) at its N terminus. This form is short-lived, as the N-terminal glutamine undergoes natural conversion to pyroglutamate, resulting in type 1 Brazzein, and the loss of the N-terminal glutamine (Q) (or pyroglutamate (pyrE)) yields the 53-amino acid type 3 Brazzein. Only the last two species are detected in the ripe fruit (approx. 80% of type 1 and approx. 20% of type 3)). The sweetness intensity varies between forms, the type 3 form is at least twice as sweet as the type 1 form. Therefore, the type 3 Brazzein is preferred. The type 1 Brazzein refers to an oligopeptide or protein of the following or amino acid sequence **(SEQ ID NO: 5):**

pyrEDKCKKVYENYPVSKCQLANQCNYDCKLDKHARSGECFYDEKRNLQCICDYCE

Y

The type 3 Brazzein refers to an oligopeptide or protein of the following amino acid sequence **(SEQ ID NO: 6):**
DKCKKVYENYPVSKCQLANQCNYDCKLDKHARSGECFYDEKRNLQCICDYCEY
The type 2 Brazzein refers to an oligopeptide or protein of the following or amino acid sequence **(SEQ ID NO: 7):**
QDKCKKVYENYPVSKCQLANQCNYDCKLDKHARSGECFYDEKRNLQCICDYCEY
The Brazzeins may be used singly or jointly in the invention. For example, type 1 and type 3 Brazzein may be used in combination. However, type 3 Brazzein is herein preferred, and the use of type 3 Brazzein as the sole Brazzein is most preferred, notably in combination with Thaumatin II as the sole Thaumatin.

The term "temporal profile" of a composition, sugar or sweetener, as used herein, is a measure of the perceived sweetness intensity of said composition, sugar or sweetener over time. A desirable or advantageous temporal profile is one wherein sweetness is observed quickly and has a short linger similar to that of sucrose.

The term "sucrose equivalent value" or "SEV" as used herein refers to the sweetness equivalent of a sweetener related to the sweetness of sucrose. For example, a sweetener at an SEV value of 5 would have a sweetness similar to a 5% by weight solution of sucrose.

The term "low calorie" as used herein refers to a sweetener having 40 calories or fewer per reference amount customarily consumed (RACC) and per labeled serving.

All amounts given in % by weight are quoted on a dry solids (ds) basis unless specifically stated otherwise.

The sweetener or flavor modifying composition comprises Thaumatin, preferably Thaumatin II, in an amount of about 5% to about 20%, and Brazzein in an amount of about 80% to about 95% by weight relative to the combined weight of the Brazzein and thaumatin portion of the composition. The Brazzein and thaumatin portion of the sweetener or flavor modifying composition makes up between about 0.00012% (1.2 ppm) to about 0.021% (210 ppm) by weight of the final food or beverage composition.

Preferably, the sweetener composition of the invention comprises Thaumatin, preferably Thaumatin II, (preferably in >90% purity) in an amount of about 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5% or 20%, and a Brazzein (preferably >90% purity) in an amount of about 80%, 80.5%, 81%, 81.5%, 82%, 82.5%, 83%, 83.5%, 84%, 84.5%, 85%, 85.5%, 86%, 86.5%, 87%, 87.5%, 88%, 88.5%, 89%, 89.5%, 90%, 90.5%, 91%, 91.5%, 92%, 92.5%, 93%, 93.5%, 94%, 94.5% or 95% by weight relative to the total weight of the Brazzein and thaumatin portion of the composition. Preferably, the sweetener or flavor modifying composition would be used in the final food or beverage composition at about 1.2 ppm, 2 ppm, 3 ppm, 4 ppm, 5 ppm, 6 ppm, 7 ppm, 8 ppm, 9 ppm, 10 ppm, 15 ppm, 20 ppm, 25 ppm, 30 ppm, 35 ppm, 40 ppm, 45 ppm, 50 ppm, 55 ppm, 60 ppm, 70 ppm, 80 ppm, 90 ppm, 100 ppm, 110 ppm, 120 ppm, 130 ppm, 140 ppm, 150 ppm, 160 ppm, 170 ppm, 180 ppm, 190 ppm, 200 ppm, or 210 ppm by weight in the final food or beverage composition.

In a preferred embodiment of the present invention, the sweetener composition comprises Thaumatin, preferably Thaumatin II, of greater than 90% purity in an amount of about 15% (preferably, about 10% to about 20%) and Brazzein of greater than 90% purity in an amount of about 85% (preferably, about 80% to about 90%) by weight relative to the total weight of the Brazzein and thaumatin portion of the composition. In another preferred embodiment of the present invention, the sweetener composition comprises Thaumatin, preferably Thaumatin II, in an amount of about 15% (preferably, about 10% to about 20%) and Brazzein in an amount of about 85% (preferably, about 80% to about 90%) by weight relative to the total weight of the Brazzein and thaumatin portion of the composition. In these embodiments, the Brazzein may be that of type 3.

In an alternative embodiment, the sweetener composition comprises Thaumatin, preferably Thaumatin II, of greater than 90% purity in an amount of about 7% (preferably, about 5% to about 10%, or about 6% to about 8%), and Brazzein in an amount of about 93% (preferably, 90% to about 95%, or about 92% to about 94%) by weight relative to the total weight of the Brazzein and thaumatin portion of the composition. In an alternative embodiment, the sweetener composition comprises Thaumatin, preferably Thaumatin II, in an amount of about 7% (preferably, about 5% to about 10%, or about 6% to about 8%), and Brazzein in an amount of about 93% (preferably, 90% to about 95%, or about 92% to about 94%) by weight relative to the total weight of the Brazzein and thaumatin portion of the composition. In these embodiments, the Brazzein may be that of type 3.

Advantageously, the sweetener composition comprises Thaumatin, preferably Thaumatin II, (preferably of a purity greater than 90%) in an amount of about 50% and Brazzein in an amount of about 50% by percentage of added sweetness in terms of relative sugar equivalent value (SEV). In an alternative embodiment, the sweetener composition comprises Thaumatin, preferably Thaumatin II, (preferably of a purity greater than 90%) in an amount of about 30% and Brazzein, preferably of a purity greater than 90%, in an amount of about 70% by percentage of added sweetness in terms of relative sugar equivalent value (SEV). In these embodiments, the Brazzein may be that of type 3. In some embodiments, the sweetener composition may further comprise a sweet taste improving additive, a bulking agent, a flavoring agent, and/or a stabilizer.

The food product comprises the sweetener or flavor modifying composition of the invention. Non-limiting examples of a food product include a confectionary product, a dessert product such as yogurt, ice-cream, biscuits, and cakes, a cereal product, baked goods, frozen dairy products, meats, dairy products, condiments, snack bars, soups, dressings, mixes, prepared foods, baby foods, diet preparations, syrups, food coatings, dried fruit, sauces, gravies, jams/jellies, and the like, especially those which are reduced sugar or low sugar products. The food product may be an animal feed product. The food product of the invention may comprise a sweetener composition as a coating or frosting formed on the surface of the product. A coating improves the flavor of the food product as well as its shelf life.

The beverage product comprises a sweetener or flavor modifying composition of the present invention. Non-limiting examples of a beverage product include a carbonated beverage, a non-carbonated beverage, fruit-flavored beverage, fruit-juice, tea, milk, coffee especially those which are reduced sugar or low sugar products.

The pharmaceutical product comprises a sweetener composition according to the invention.

The nutritional product comprise a sweetener composition according to the invention.

The cosmetic product comprises a sweetener composition according to the invention.

It will be appreciated that the amount of a sweetener composition according to the invention present in a food product, a beverage product, a pharmaceutical product, a nutritional product, or a cosmetic product, will depend upon the type and amount of sweetener present in the sweetener composition and the desired sweetness of the food or beverage product.

The sweetener or flavor modifying composition according to the invention may be used in a food product, a beverage product, a pharmaceutical product, a nutritional product, a sports product, or a cosmetic product, as a bulking agent or as a coating agent.

The sweetener or flavor modifying composition may be formulated in any physical form, for example, dissolved in a solvent such as water or glycol, as a syrup, in powder form, tablet form, as granules, in a solution or in any other suitable form including beverages and food products.

As outlined in the below examples, the sweetener composition of the invention exhibits a sucrose equivalent value (SEV) greater than the predicted value based on its individual components. Therefore, the sweetener composition of the present invention displays sweetness synergy.

EXAMPLES

The following examples are exemplary only and is not intended to be limiting in any way.

**Production Example:** Method of expressing Brazzein Translational fusion of the sequence **(SEQ ID NO: 9)** encoding N-terminal apoplast targeting presequence from Oryza sativa RAmy3A gene for alpha-amylase and coding sequence for mature Brazzein type 3 **(SEQ ID No:** 8) followed by stop-codon were inserted in pNMD035 plasmid resulting in pNMD52701 construct **(Fig. 1). SEQ ID NO: 10** is nucleotide sequence of T-DNA region of pNMD52701 vector.

Coding sequence of the type 3 Brazzein refers to the following nucleotide sequence **(SEQ ID NO: 8):**

gacaagtgcaagaaggtctacgagaactacccggtgtccaagtgccagctggccaaccagtgcaactacgactgcaagctggacaa

gcacgcccgctccggcgagtgcttctacgacgagaagcgcaacctgcagtgcatctgcgactactgcgagtactaa

Translational fusion of the type 3 Brazzein with apoplast targeting presequence from rice amylase refers to the following nucleotide sequence **(SEQ ID NO: 9):**

Atggggaagcaaatggccgccctgtgtggctttctcctcgtggcgttgctctggctcacgcccgacgtcgcgtcaggtgacaagtgc

aagaaggtctacgagaactacccggtgtccaagtgccagctggccaaccagtgcaactacgactgcaagctggacaagcacgccc

gctccggcgagtgcttctacgacgagaagcgcaacctgcagtgcatctgcgactactgcgagtactaa

*Nicotiana benthamiana* plants were grown in the greenhouse and transfected with *Agrobacterium tumefacience* cells carrying pNMD52701 construct using vacuum infiltration as described in WO 2022/012926 A1 for Thaumatin. Plant biomass was harvested at 7 days post infiltration (7 dpi).

Brazzein-containing plant biomass was extracted with 20 mM Na-acetate pH 4.0, 30 mM NaCl with a buffer-to-biomass ratio of 3:1 using a juicer. Green juice was incubated overnight at 58°C. The extract was then filtrated through Miracloth and filter press with 3 $\mu$m pore size (8 filters) and further cleared using 0.7 $\mu$m filter.

Cleared extract was further purified using column chromatography on Äkta System with CaptoMMC resin (Cytiva). The column was equilibrated with the extraction buffer. Washing was performed using the extraction buffer (wash 1), 15% elution buffer (wash 2) and 35% elution buffer (wash 3). The elution was performed with 100% elution buffer (100 mM NaH$_2$PO$_4$ pH 7.5, 10 mM NaCl).

Pooled Brazzein fractions were further purified using column chromatography with HiTrapQ FF resin (Cytiva). The column was equilibrated with 31 mM NaH$_2$PO$_4$ pH 7.5, 3.1 mM NaCl. Column was washed with 20 mM Na-acetate pH 4.0, 30 mM NaCl. Flowthrough and wash fractions were collected and combined together as these fractions contained Brazzein (negative chromatography). The column was further eluted with 10 mM NaH$_2$PO$_4$, pH 7.5, 1 M NaCl. Elution fractions were discarded.

Pooled Brazzein-containing fractions were desalted overnight using 1kDa Minimate™ FF membrane (Pall). Brazzein solution was further sterile filtrated through 0.22 $\mu$m bottle top filter. The protein concentrated was determined using A280-method. The aliquots were further analyzed for purity and integrity using SDS-PAGE, Capillary Gel Electrophoresis and Mass Spectrometry methods. Brazzein solution was freeze-dried for storage.

**Example 1:** Demonstration of Sweetness Synergy of the Composition of the Present Invention.

Round table evaluations were performed with 6 test panelists. Equal sweet 5 SEV concentrations in neutral pH water were made for Thaumatin II (purity greater than 90%), and Brazzein. These equal sweet solutions were also mixed at 50% of Thaumatin II and 50% Brazzein and compared to the individual components. The components of the test compositions are described in the below tables. The mixed compositions were calculated using the Beidler mixture equation for the sweeteners. The Beidler mixture equation for sweeteners is as follows:

$$SEV = \frac{conc \cdot R_{max}}{conc + 1/K}$$

The concentration of each component in the mixture in ppm is divided by SEV (c/R) and is plotted against concentration, c. The slope of the linear regression is the maximum SEV ($R_{max}$). The y-intercept of the linear regression multiplied by Rmax is the half-maximal sweetness concentration, 1/K. $R_{max}$ and 1/K are the two parameters used in the Beidler equation. Mixtures were tasted in comparison to reference samples for the panelists to determine SEV values. References samples were 3%, 4%, 5%, 6%, 7%, and 8% sucrose in neutral pH water. The test samples were served in 2 ounce soufflé cups (approximately 60 ml) coded with 3-digit codes at room temperature. A two minute wait period between samples was enforced. Water and unsalted crackers was available for the panelists to clear their palates before and during testing. Results were collected for calculating approximate SEV level of each test sample.
The test products analyzed in this experiment are described below in Table 1.

Product Information

TABLE 1. The mean sweetness for Brazzein, Thaumatin II, and the combination of Brazzein and Thaumatin II.

| Nr | Composition | Sweetness intensity determined, SEV |
|---|---|---|
| 1 | 7 ppm Thaumatin II | 5.1 |
| 2 | 30 ppm Brazzein | 5.4 |
| 3 | 3.5 ppm Thaumatin II / 15 ppm Brazzein | 5.9 |

Surprisingly combinations of Thaumatin II and Brazzein provided sweeter solutions than the individual components would suggest.

Example 2 Qualitative analysis of individual sweeteners and Round table evaluations were performed with test panelists. Equal sweet 5 SEV concentrations in neutral pH water were made for Thaumatin II (purity greater than 90%), and Brazzein (purity greater than 90%). These equal sweet solutions were also mixed at 50% of Thaumatin II and 50% Brazzein and compared to the individual components. All solutions were coded and blinded from panelists. Panelists were asked to provide a qualitative assessment on a scale of 1-10 of the quality of sweetness (10 being the highest quality of sweetness) and undesirable attributes (10 being most undesirable) of each solution using a 5% sucrose solution as a control.

TABLE 2. the mean taste quality of Thaumatin II, Brazzein, and the combination of Brazzein and Thaumatin II.

| Sample | Quality of sweetness (higher better) | Off taste (lower better) | Comments |
|---|---|---|---|
| 7 ppm Thaumatin II | 5.2 | 5.4 | Delayed onset, sweet aftertaste |
| 30 ppm Brazzein | 7.7 | 2.3 | Rapid onset, no pronounced off taste |
| 3.5 ppm Thaumatin II / 15 ppm Brazzein | 6.8 | 4.8 | No delay, slight sweet aftertaste |

Surprisingly, mixtures of Thaumatin II and Brazzein had significantly better quality of sweetness and reduced off taste.

Example 3: Protein sweetener labeling. Mixtures of various sweeteners were combined and assessed for their ability to maintain a high quality of sweetness and be sweetened solely by protein sweeteners, and sweeteners of natural origin.

**TABLE 3**.Tthe sweetness quality of various sweeteners combined and assessed for their ability to maintain a high quality of sweetness.

| Sweetener | Quality of Sweetness | Off Taste | Protein Sweetener Only? | Natural Origin | Comments |
|---|---|---|---|---|---|
| 3.5 ppm Thaumatin II / 145 ppm Aspartame | 5.3 | 6.4 | No | No | Artificial off taste |
| 7 ppm Thaumatin II | 5.2 | 5.4 | Yes | Yes | Delayed onset, sweet aftertaste |
| 30 ppm Brazzein | 7.7 | 2.3 | Yes | Yes | Rapid onset, no pronounced off taste |
| 7 ppm Thaumatin II / 30 ppm Brazzein | 6.8 | 3.8 | Yes | Yes | No delay, slight sweet aftertaste |
| 15 ppm Brazzein / 77.5 ppm Rebaudioside A | 6.1 | 6.4 | No | Yes | Artificial off taste |
| 145 ppm Aspartame / 145 ppm Acesulfame K | 5.9 | 6.6 | No | No | Artificial off taste |
| 48 ppm Sucralose / 145 ppm Acesulfame K | 7.9 | 2.2 | No | No | Clean sweet taste |

We demonstrated that protein sweeteners provide the sweetness quality equal or superior the sweetness quality of various commercial artificial or natural sweeteners.

**Example 4.** Analysis of total sweet perception for Thaumatin II, Brazzein and their mixture

Recombinant Thaumatin II **(SEQ ID NO: 2)** and Brazzein type 3 **(SEQ ID NO: 6)** proteins were purified from *Nicotiana* plants transgenic for ethanol-inducible replicons of Tobacco Mosaic Virus carrying corresponding coding sequences. Recombinant protein expression was achieved by spraying plants with diluted ethanol solution. Thaumatin II protein was purified from plant biomass using column chromatography. For Thaumatin II production, the details of upstream and downstream processes are described in WO 2022/012926 A1. Brazzein protein was produced in a similar way, as described in the Production Example.

7 ppm recombinant Thaumatin II and 30 ppm Brazzein water solutions as well as both of them mixed together in the half-concentrations (3.5 ppm Thaumatin II / 15 ppm Brazzein) were analysed for the total sweet perception. 5% sucrose solution was used as a sweetness control. Flavor and aftertaste were assessed by 8 members of the trained sensory panel of Brisan Group (Orland Park, IL, USA) trained in the Spectrum descriptive methodology. The sweetness intensity was rated on the 15-point Spectrum Scale, where 0 = none and 15 = very strong. Products were evaluated at room temperature. Products were blinded with 3-digit codes and randomized. Breaks were given in between samples to reduce fatigue. All references were available to panelists to determine intensity scores. Flavour and aftertaste data was collected as individual data. All samples were expectorated. Samples were presented blind in a monadic sequential balanced design. Two replications of sample data were collected.

Panelists evaluated the total sweetness intensity immediately after starting the exposure to test solutions (0-5 seconds in the mouth) and at 15 seconds while the product was still in the mouth. Panelists also evaluated the aftertaste of products 30 seconds and 60 seconds after evaluating the product in mouth (after the expectoration).

To analyse the immediate sweet perception, panelists evaluated the sweet intensity immediately after taking a sip of the solution (evaluated within 0-5 seconds). The total sweetness of products was defined as a sum of the sweet basic taste and high intensity sweetener aromatic attribute. The sweet basic taste was defined as the taste on the tongue stimulated by sucrose and other sugars, such as fructose, glucose, etc. High intensity sweetener aromatic was defined as the light aromatic that is inherent with any non-sucrose sweetener, such as Aspartame, Ace-K, Reb A, Stevia, and Sucralose.

**Figure 2** summarizes the data on the total mean sweet perception for Thaumatin II, Brazzein, and the combination thereof. A higher total sweetness was found for the mix compared to what one would have expected for the additive sweetness of the individual components, each at the concentration as used in the mix. This effect is more pronounced beginning 15th second. This data is supportive for the synergistic effect between Thaumatin II and Brazzein. Brazzein also makes the sweetness onset of Thaumatin II less steep, thus providing improved sweetness temporal profile to the mixture.

**CONCLUSIONS**

A sweetener or flavor modifying composition according to the present invention exhibits a sweetness quality of high acceptability, is composed entirely of protein sweeteners of natural origin, and has an acceptable cost in use, whereas no other blend of sweeteners conceived has these attributes. Surprisingly, the sweetener or flavor modifying composition of the present invention also has significant sweetness synergy in addition to a substantial improvement in taste quality and concomitant reduction in undesirable organoleptic properties.

FURTHER SEQUENCE LISTING

**SEQ ID NO: 10** is the nucleotide sequence of the T-DNA region of pNMD52701 vector.
cctgtggttggcacatacaaatggacgaacggataaaccttttcacgcccttttaaatatccgattattctaataaacgctcttttctcttagg
tttacccgccaatatatcctgtcaaacactgatagtttaaactgaaggcgggaaacgacaatctgatctaagctagcttggaattggtacc
acgcgtttcgacaaaatttagaacgaacttaattatgatctcaaatacattgatacatatctcatctagatctaggttatcattatgtaagaaa
gttttgacgaatatggcacgacaaaatggctagactcgatgtaattggtatctcaactcaacattatacttataccaaacattagttagaca

aaatttaaacaactatttttttatgtatgcaagagtcagcatatgtataattgattcagaatcgttttgacgagttcggatgtagtagtagccatt
atttaatgtacatactaatcgtgaatagtgaatatgatgaaacattgtatcttattgtataaatatccataaacacatcatgaaagacactttct
ttcacggtctgaattaattatgatacaattctaatagaaaacgaattaaattacgttgaattgtatgaaatctaattgaacaagccaaccacg
acgacgactaacgttgcctggattgactcggtttaagttaaccactaaaaaaacggagctgtcatgtaacacgcggatcgagcaggtc
acagtcatgaagccatcaaagcaaaagaactaatccaagggctgagatgattaattagtttaaaaattagttaacacgagggaaaagg
ctgtctgacagccaggtcacgttatctttacctgtggtcgaaatgattcgtgtctgtcgattttaattattttttgaaaggccgaaaataaagt
tgtaagagataaacccgcctatataaattcatatattttcctctccgctttgaagttttagtttattgcaacaacaacaacaaattacaataac
aacaaacaaaatacaaacaacaacaacatggcacaatttcaacaaacaattgacatgcaaactctccaagccgctgcgggacgcaac
agcttggtgaatgatttggcatctcgtcgcgtttacgataatgcagtcgaggagctgaatgctcgttccagacgtcccaaggtaatagga
actttctggatctactttatttgctggatctcgatcttgttttctcaatttccttgagatctggaattcgtttaatttggatctgtgaacctccacta
aatcttttggttttactagaatcgatctaagttgaccgatcagttagctcgattatagctaccagaatttggcttgaccttgatggagagatcc
atgttcatgttacctgggaaatgatttgtatatgtgaattgaaatctgaactgttgaagttagattgaatctgaacactgtcaatgttagattga
atctgaacactgtttaaggttagatgaagtttgtgtatagattcttcgaaactttaggatttgtagtgtcgtacgttgaacagaaagctatttct
gattcaatcagggtttatttgactgtattgaactcttttgtgtgtttgcaggtccacttctccaaggcagtgtctacggaacagaccctgatt
gcaacaaacgcatatccggagttcgagatttcctttactcatacgcaatccgctgtgcactccttggccggaggccttcggtcacttgag
ttggagtatctcatgatgcaagttccgttcggtctctgacgtacgacatcggcggtaacttttccgcgcacctttcaaagggcgcgatta
cgttcactgctgcatgcctaatctggatgtacgtgacattgctcgccatgaaggacacaaggaagctatttacagttatgtgaatcgtttg
aaaaggcagcagcgtcctgtgcctgaataccagagggcagctttcaacaactacgctgagaacccgcacttcgtccattgcgacaaa
cctttccaacagtgtgaattgacgacagcgaatggcactgacacctacgctgtagctctccatagcatttatgatatccctgttgaggagt
tcggttctgcgctactcaggaagaatgtgaaaacttgtttcgcggcctttcatttccatgagaatatgcttctagattgtgatacagtcacac
tcgatgagattggagctacgttccagaaatcaggtaacattccttagttacctttcttttcttttccatcataagtttatagattgtacatgcttt
gagattttctcttgcaaacaatctcaggtgataacctgagcttcttcttccataatgagagcactctcaattacacccacagcttcagcaaca
tcatcaagtacgtgtgcaagacgttcttccctgctagtcaacgcttcgtgtaccacaaggagttcctggtcactagagtcaacacttggta
ctgcaagttcacgagagtggatacgttcactctgttccgtggtgtgtaccacaacaatgtggattgcgaagagttttacaaggctatgga
cgatgcgtggcactacaaaaagacgttagcaatgcttaatgccgagaggaccatcttcaaggataacgctgcgttaaacttctggttcc
cgaaggtgctcttgaaattggaagtcttcttttgttgtctaaacctatcaatttctttgcggaaatttatttgaagctgtagagttaaaattgagt
ctttttaaacttttgtaggtgagagacatggttatcgtccctctctttgacgcttctatcacaactggtaggatgtctaggagagaggttatgg
tgaacaaggacttcgtctacacggtcctaaatcacatcaagacctatcaagctaaggcactgacgtacgcaaacgtgctgagcttcgtg
gagtctattaggtctagagtgataattaacggtgtcactgccaggtaagttgttacttatgattgtttttcctctctgctacatgtattttgttgttc
atttctgtaagatataagaattgagttttcctctgatgatattattaggtctgaatgggacacagacaaggcaattctaggtccattagcaat
gacattcttcctgatcacgaagctgggtcatgtgcaagatgaaataatcctgaaaaagttccagaagttcgacagaaccaccaatgagc
tgatttggacaagtctctgcgatgccctgatggggttattccctcggtcaaggagacgcttgtgcgcggtggttttgtgaaagtagcag
aacaagccttagagatcaaggttagtatcatatgaagaaatacctagtttcagttgatgaatgctattttctgacctcagttgttctcttttgag
aattatttcttttctaatttgcctgattttctattaattcattaggttcccgagctatactgtaccttcgccgaccgattggtactacagtacaag
aaggcggaggagttccaatcgtgtgatctttccaaacctctagaagagtcagagaagtactacaacgcattatccgagctatcagtgctt
gagaatctcgactcttttgacttagaggcgtttaagacttatgtcagcagaagaatgtggacccggatatggcagcaaaggtaaatcct
ggtccacacttttacgataaaaacacaagatttaaactatgaactgatcaataatcattcctaaaagaccacacttttgttttgtttctaaagt
aattttactgttataacaggtggtcgtagcaatcatgaagtcagaattgacgttgcctttcaagaaacctacagaagaggaaatctcgga
gtcgctaaaaccaggagaggggtcgtgtgcagagcataaggaagtgttgagcttacaaaatgatgctccgttcccgtgtgtgaaaaat
ctagttgaaggttccgtgccggcgtatggaatgtgtcctaagggtggtggtttcgacaaattggatgtggacattgctgatttccatctca
agagtgtagatgcagttaaaaagggaactatgatgtctgcggtgtacacagggtctatcaaagttcaacaaatgaagaactacatagatt
acttaagtgcgtcgctggcagctacagtctcaaacctctgcaaggtaagaggtcaaaaggtttccgcaatgatccctctttttttgtttctct
agtttcaagaatttgggtatatgactaacttctgagtgttccttgatgcatatttgtgatgagacaaatgtttgttctatgttttaggtgcttaga
gatgttcacggcgttgacccagagtcacaggagaaatctggagtgtgggatgttaggagaggacgttggttacttaaacctaatgcga
aaagtcacgcgtggggtgtggcagaagacgccaaccacaagttggttattgtgttactcaactgggatgacggaaagccggtttgtga
tgagacatggttcagggtggcggtgtcaagcgattccttgatatattcggatatgggaaaacttaagacgctcacgtcttgcagtccaaa
tggtgagccaccggagcctaacgccaaagtaattttggtcgatggtgttcccggttgtggaaaaacgaaggagattatcgaaaggta
agttctgcatttggttatgctccttgcatttttaggtgttcgtcgctcttccatttccatgaatagctaagatttttttctctgcattcattcttcttgc
ctcagttctaactgtttgtggtatttttgttttaattattgctacaggtaaacttctctgaagacttgattttagtccctgggaaggaagcttctaa
gatgatcatccggagggccaaccaagctggtgtgataagagcggataaggacaatgttagaacggtggattccttcttgatgcatcctt

ctagaagggtgtttaagaggttgtttatcgatgaaggactaatgctgcatacaggttgtgtaaatttcctactgctgctatctcaatgtgacg
tcgcatatgtgtatggggacacaaagcaaattccgttcatttgcagagtcgcgaactttccgtatccagcgcattttgcaaaactcgtcgc
tgatgagaaggaagtcagaagagttacgctcaggtaaagcaactgtgttttaatcaatttcttgtcaggatatatggattataacttaatttt
gagaaatctgtagtatttggcgtgaaatgagtttgcttttggtttctcccgtgttataggtgcccggctgatgttacgtatttccttaacaaga
agtatgacggggcggtgatgtgtaccagcgcggtagagagatccgtgaaggcagaagtggtgagaggaaagggtgcattgaaccc
aataaccttaccgttggagggtaaaattttgaccttcacacaagctgacaagttcgagttactggagaagggttacaaggtaaagtttcc
aactttcctttaccatatcaaactaaagttcgaaacttttatttgatcaacttcaaggccacccgatctttctattcctgattaatttgtgatgaa
tccatattgacttttgatggttacgcaggatgtgaacactgtgcacgaggtgcaaggggagacgtacgagaagactgctattgtgcgctt
gacatcaactccgttagagatcatatcgagtgcgtcacctcatgttttggtggcgctgacaagacacacaacgtgttgtaaatattacacc
gttgtgttggacccgatggtgaatgtgatttcagaaatggagaagttgtccaatttccttcttgacatgtatagagttgaagcaggtctgtct
ttcctatttcatatgtttaatcctaggaatttgatcaattgattgtatgtatgtcgatcccaagactttcttgttcacttatatcttaactctctcttg
ctgtttcttgcaggtgtccaatagcaattacaaatcgatgcagtattcaggggacagaacttgtttgttcagacgcccaagtcaggagatt
ggcgagatatgcaattttactatgacgctcttcttcccggaaacagtactattctcaatgaatttgatgctgttacgatgaatttgagggatat
ttccttaaacgtcaaagattgcagaatcgacttctccaaatccgtgcaacttcctaaagaacaacctattttcctcaagcctaaaataagaa
ctgcggcagaaatgccgagaactgcaggtaaaatattggatgccagacgatattctttcttttgatttgtaacttttcctgtcaaggtcgat
aaattttattttttttggtaaaaggtcgataattttttttggagccattatgtaattttcctaattaactgaaccaaaattatacaaaccaggtttg
ctggaaaatttggttgcaatgatcaaaagaaacatgaatgcgccggatttgacagggacaattgacattgaggatactgcatctctggtg
gttgaaaagttttgggattcgtatgttgacaaggaatttagtggaacgaacgaaatgaccatgacaagggagagcttctccaggtaagg
acttctcatgaatattagtggcagattagtgttgttaaagtctttggttagataatcgatgcctcctaattgtccatgttttactggttttctacaa
ttaaaggtggctttcgaaacaagagtcatctacagttggtcagttagcggactttaactttgtggatttgccggcagtagatgagtacaag
catatgatcaagagtcaaccaaagcaaaagttagacttgagtattcaagacgaatatcctgcattgcagacgatagtctaccattcgaaa
aagatcaatgcgattttcggtccaatgttttcagaacttacgaggatgttactcgaaaggattgactcttcgaagtttctgttctacaccaga
aagacacctgcacaaatagaggacttctttctgacctagactcaacccaggcgatggaaattctggaactcgacatttcgaagtacgat
aagtcacaaaacgagttccattgtgctgtagagtacaagatctgggaaaagttaggaattgatgagtggctagctgaggtctggaaaca
aggtgagttcctaagttccatttttttgtaatccttcaatgttattttaacttttcagatcaacatcaaaattaggttcaattttcatcaaccaaata
atattttttcatgtatatataggtcacagaaaaacgaccttgaaagattatacggccggaatcaaaacatgtctttggtatcaaaggaaaagt
ggtgatgtgacaacctttattggtaataccatcatcattgccgcatgtttgagctcaatgatccccatggacaaagtgataaaggcagcttt
ttgtggagacgatagcctgatttacattcctaaaggtttagacttgcctgatattcaggcgggcgcgaacctcatgtggaacttcgaggc
caaactcttcaggaagaagtatggttacttctgtggtcgttatgttattcaccatgatagaggagccattgtgtattacgatccgcttaaact
aatatctaagttaggttgtaaacatattagagatgttgttcacttagaagagttacgcgagtctttgtgtgatgtagctagtaacttaaataatt
gtgcgtattttttcacagttagatgaggccgttgccgaggttcataagaccgcggtaggcggttcgtttgcttttgtagtataattaagtattt
gtcagataagagattgtttagagatttgttctttgtttgataatgtcgatagtctcgtacgaacctaaggtgagtgatttcctcaatctttcgaa
gaaggaagagatcttgccgaaggctctaacgaggttaaaaaccgtgtctattagtactaaagatattatatctgtcaaggagtcggaga
ctttgtgtgatatagatttgttaatcaatgtgccattagataagtatagatatgtgggtatcctaggagccgttttaccggagagtggctagt
gccagacttcgttaaaggtggagtgacgataagtgtgatagataagcgtctggtgaactcaaaggagtgcgtgattggtacgtacaga
gccgcagccaagagtaagaggttccagttcaaattggttccaaattactttgtgtccaccgtggacgcaaagaggaagccgtggcagg
taaggattttttatgatatagtatgcttatgtattttgtactgaaagcatatcctgcttcattgggatattactgaaagcatttaactacatgtaaa
ctcacttgatgatcaataaacttgattttgcaggttcatgttcgtatacaagacttgaagattgaggcgcgggttggcagccgttagctctgga
agtagtttcagttgctatggtcaccaataacgttgtcatgaagggtttgagggaaaaggtcgtcgcaataaatgatccggacgtcgaag
gtttcgaaggtaagccatcttcctgcttatttttataatgaacatagaaataggaagttgtgtcagagaaactaattaacctgactcaaaatct
accctcataattgttgtttgatattggtcttgtattttgcaggtgtggttgacgaattcgtcgattcggttgcagcatttaaagcggttgacaac
tttaaaagaaggaaaaagaaggttgaagaaaagggtgtagtaagtaagtataagtacagaccggagaagtacgccggtcctgattcgt
ttaatttgaaagaagaaaacgtcttacaacattacaaacccgaatcagtaccagtatttcgataagctagcaaacaagaaatggggaag
caaatggccgccctgtgtggctttctcctcgtggccgttgctctggctcacgcccgacgtcgcgtcaggtgacaagtgcaagaaggtcta
cgagaactacccggtgtccaagtgccagctggccaaccagtgcaactacgactgcaagctggacaagcacgcccgctccggcgag
tgcttctacgacgagaagcgcaacctgcagtgcatctgcgactactgcgagtactaagcttactagagcgtggtgcgcacgatagcgc
atagtgttttctctccacttgaatcgaagagatagacttacggtgtaaatccgtagggggtggcgtaaaccaaattacgcaatgtttgggt
tccatttaaatcgaaaccccttatttcctggatcacctgttaacgcacgtttgacgtgtattacagtgggaataagtaaaagtgagaggttc
gaatcctccctaaccccgggtagggg

**Claims**

1. A sweetener or flavor modifying composition comprising a Thaumatin and a Brazzein, said composition comprising Thaumatin in an amount of about 5% to about 20% and Brazzein in an amount of about 80% to about 95% by weight relative to the combined weight of the Brazzein and Thaumatin portion of the composition.

2. The composition according to claim 1, wherein the Thaumatin is selected from Thaumatin I, Thaumatin II, Thaumatin A, and Thaumatin B; and/or wherein the Brazzein is selected from type 1 Brazzein and type 3 Brazzein.

3. The composition according to claim 1 or 2, wherein the Thaumatin is Thaumatin I or Thaumatin II and the Brazzein is selected from type 1 Brazzein and type 3 Brazzein, preferably the Thaumatin is Thaumatin II, more preferably the Thaumatin is Thaumatin II and the Brazzein is type 3 Brazzein.

4. The sweetener or flavor modifying composition according to any one of claims 1 to 3, wherein the Thaumatin is not isolated from *Thamautococcus Daniellii,* and/or wherein the Brazzein is not isolated from *Pentadiplandra brazzeana Baillon.*

5. The sweetener or flavor modifying composition according to any one of claims 1 to 4, wherein the sweetener composition comprises Thaumatin, preferably Thaumatin II, in an amount of 10% to about 20% and Brazzein in an amount of about 80% to about 90% by weight relative to the total weight of the Brazzein and Thaumatin of the composition.

6. The sweetener or flavor modifying composition of any one of claims 1-5, wherein the composition contains less than 0.001% of any proteins originating from either *Thamatococcus* or *Pantadiplandra* genus organisms excluding Thaumatin or Brazzein.

7. The sweetener or flavor modifying composition according to any one of claims 1 to 4, comprising, as said Thaumatin, Thaumatin II of greater than 90% purity and, as said Brazzein, Brazzein of greater than 90% purity.

8. The sweetener or flavor modifying composition according to any one of claims 1 to 4, comprising Thaumatin, preferably Thaumatin II, in an amount of about 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5% or 20%, and a Brazzein in an amount of about 90%, 90.5%, 91%, 91.5%, 92%, 92.5%, 93%, 93.5%, 94%, 94.5% or 95% by weight relative to the total weight of the Brazzein and thaumatin of the composition.

9. A method of manufacturing a reduced-calorie product, comprising adding the sweetener or flavor modifying composition according to any one of claims 1 to 8 to a food product, a beverage product, a pharmaceutical product, a nutritional product, a sports product, or a cosmetic product.

10. A food, beverage, nutritional product, cosmetic product, or pharmaceutical product comprising the sweetener or flavor modifying composition of any one of claims 1 to 8 at a use level between about 1.2 ppm and about 210 ppm.

11. The food, beverage, nutritional product, cosmetic product, or pharmaceutical product according to claim 10, comprising the sweetener or flavor modifying composition at a use level about 40 ppm or at a use level about 10 ppm.

**Patentansprüche**

1. Süßstoffzusammensetzung oder geschmacksmodifizierende Zusammensetzung, umfassend Thaumatin und Brazzein, wobei die Zusammensetzung Thaumatin in einer Menge von etwa 5 % bis etwa 20 % und Brazzein in einer Menge von etwa 80 % bis etwa 95 Gewichts-% bezogen auf das Gesamtgewicht des Brazzein- und Thaumatinanteils der Zusammensetzung enthält.

2. Die Zusammensetzung gemäß Anspruch 1, wobei das Thaumatin aus Thaumatin I, Thaumatin II, Thaumatin A und Thaumatin B ausgewählt ist; und/oder wobei das Brazzein aus Typ-1-Brazzein und Typ-3-Brazzein ausgewählt ist.

3. Die Zusammensetzung gemäß Anspruch 1 oder 2, wobei das Thaumatin Thaumatin I oder Thaumatin II ist und das Brazzein aus Typ-1-Brazzein und Typ-3-Brazzein ausgewählt ist, vorzugsweise ist das Thaumatin Thaumatin II, noch bevorzugter ist das Thaumatin Thaumatin II und das Brazzein ist Typ-3-Brazzein.

4. Die Süßstoffzusammensetzung oder geschmacksmodifizierende Zusammensetzung gemäß einem der Ansprüche 1 bis 3, wobei das Thaumatin nicht aus *Thamautococcus Daniellii* isoliert ist und/oder wobei das Brazzein nicht aus *Pentadiplandra brazzeana Baillon* isoliert ist.

5. Die Süßstoffzusammensetzung oder geschmacksmodifizierende Zusammensetzung gemäß einem der Ansprüche 1 bis 4, wobei die Süßungsmittelzusammensetzung Thaumatin, vorzugsweise Thaumatin II, in einer Menge von 10 % bis etwa 20 % und Brazzein in einer Menge von etwa 80 % bis etwa 90 % Gewichts-% bezogen auf das Gesamtgewicht des Brazzeins und Thaumatins der Zusammensetzung, umfasst.

6. Die Süßstoffzusammensetzung oder geschmacksmodifizierende Zusammensetzung nach einem der Ansprüche 1 bis 5, wobei die Zusammensetzung weniger als 0,001 % an Proteinen enthält, die aus Organismen der Gattung *Thamatococcus* oder *Pantadiplandra* stammen, mit Ausnahme von Thaumatin oder Brazzein.

7. Die Süßstoffzusammensetzung oder geschmacksmodifizierende Zusammensetzung nach einem der Ansprüche 1 bis 4, umfassend Thaumatin II als Thaumatin mit einer Reinheit von mehr als 90 % und als Brazzein Brazzein mit einer Reinheit von mehr als 90 %.

8. Die Süßstoffzusammensetzung oder geschmacksmodifizierende Zusammensetzung gemäß einem der Ansprüche 1 bis 4, umfassend Thaumatin, vorzugsweise Thaumatin II, in einer Menge von etwa 10 %, 10,5 %, 11 %, 11,5 %, 12 %, 12,5 %, 13 %, 13,5 %, 14 %, 14,5 %, 15 %, 15,5 %, 16 %, 16,5 %, 17 %, 17,5 %, 18 %, 18,5 %, 19 %, 19,5 % oder 20 % und ein Brazzein in einer Menge von etwa 90 %, 90,5 %, 91 %, 91,5 %, 92 %, 92,5 %, 93 %, 93,5 %, 94 %, 94,5 % oder 95 Gewichts-% bezogen auf das Gesamtgewicht des Brazzeins und Thaumatins der Zusammensetzung.

9. Verfahren zur Herstellung eines kalorienreduzierten Produkts, umfassend das Hinzufügen der Süßstoffzusammensetzung oder geschmacksmodifizierende Zusammensetzung gemäß einem der Ansprüche 1 bis 8 zu einem Lebensmittelprodukt, einem Getränkeprodukt, einem pharmazeutischen Produkt, einem Nahrungsergänzungsmittel, einem Sportprodukt oder einem Kosmetikprodukt.

10. Lebensmittel, Getränk, Nahrungsergänzungsmittel, Kosmetikprodukt oder pharmazeutisches Produkt, umfassend die Süßstoffzusammensetzung oder geschmacksmodifizierende Zusammensetzung nach einem der Ansprüche 1 bis 8 in einer Anwendungsmenge zwischen etwa 1,2 ppm und etwa 210 ppm.

11. Lebensmittel, Getränk, Nahrungsergänzungsmittel, Kosmetikprodukt oder pharmazeutisches Produkt gemäß Anspruch 10, umfassend die Süßstoffzusammensetzung oder geschmacksmodifizierende Zusammensetzung in einer Anwendungsmenge von etwa 40 ppm oder in einer Anwendungsmenge von etwa 10 ppm.

## Revendications

1. Composition édulcorante ou modificatrice d'arôme comprenant une thaumatine et une brazzéine, ladite composition comprenant la thaumatine en une quantité d'environ 5 % à environ 20 % et la brazzéine en une quantité d'environ 80 % à environ 95 % en poids par rapport au poids combiné de la partie de brazzéine et de thaumatine de la composition.

2. Composition selon la revendication 1, dans laquelle la thaumatine est sélectionnée parmi la thaumatine I, la thaumatine II, la thaumatine A et la thaumatine B ; et/ou dans laquelle la brazzéine est sélectionnée parmi la brazzéine de type 1 et la brazzéine de type 3.

3. Composition selon la revendication 1 ou 2, dans laquelle la thaumatine est la thaumatine I ou la thaumatine II et la brazzéine est sélectionnée parmi la brazzéine de type 1 et la brazzéine de type 3, de préférence la thaumatine est la thaumatine II, de manière davantage préférée la thaumatine est la thaumatine II et la brazzéine est la brazzéine de type 3.

4. Composition édulcorante ou modificatrice d'arôme selon l'une des revendications 1 à 3, dans laquelle la thaumatine n'est pas isolée de *Thamautococcus Daniellii* et/ou dans laquelle la brazzéine n'est pas isolée de *Pentadiplandra*

*brazzeana Baillon.*

5. Composition édulcorante ou modificatrice d'arôme selon l'une des revendications 1 à 4, dans laquelle la composition édulcorante comprend de la thaumatine, de préférence de la thaumatine II, en une quantité de 10 % à environ 20 % et de la brazzéine en une quantité d'environ 80 % à environ 90 % en poids par rapport au poids total de la brazzéine et de la thaumatine de la composition.

6. Composition édulcorante ou modificatrice d'arôme selon l'une des revendications 1 à 5, dans laquelle la composition contient moins de 0,001 % de protéines provenant d'organismes du genre *Thamatococcus* ou *Pantadiplandra* à l'exclusion de la thaumatine ou de la brazzéine.

7. Composition édulcorante ou modificatrice d'arôme selon l'une des revendications 1 à 4, comprenant, comme ladite thaumatine, de la thaumatine II d'une pureté supérieure à 90 % et, comme ladite brazzéine, de la brazzéine d'une pureté supérieure à 90 %.

8. Composition édulcorante ou modificatrice d'arôme selon l'une des revendications 1 à 4, comprenant de la thaumatine, de préférence de la thaumatine II, en une quantité d'environ 10 %, 10,5 %, 11 %, 11,5 %, 12 %, 12,5 %, 13 %, 13,5 %, 14 %, 14,5 %, 15 %, 15,5 %, 16 %, 16,5 %, 17 %, 17,5 %, 18 %, 18,5 %, 19 %, 19,5 % ou 20 %, et une brazzéine en une quantité d'environ 90 %, 90,5 %, 91 %, 91,5 %, 92 %, 92,5 %, 93 %, 93,5 %, 94 %, 94,5 % ou 95 % en poids par rapport au poids total de la brazzéine et de la thaumatine de la composition.

9. Procédé de fabrication d'un produit à faible teneur en calories, comprenant l'ajout de la composition édulcorante ou modificatrice d'arôme selon l'une des revendications 1 à 8 à un produit alimentaire, un produit de boisson, un produit pharmaceutique, un produit nutritionnel, un produit de sport ou un produit cosmétique.

10. Aliment, boisson, produit nutritionnel, produit cosmétique ou produit pharmaceutique comprenant la composition édulcorante ou modificatrice d'arôme selon l'une des revendications 1 à 8 à un niveau d'utilisation compris entre environ 1,2 ppm et environ 210 ppm.

11. Aliment, boisson, produit nutritionnel, produit cosmétique ou produit pharmaceutique selon la revendication 10, comprenant la composition édulcorante ou modificatrice d'arôme à un niveau d'utilisation d'environ 40 ppm ou à un niveau d'utilisation d'environ 10 ppm.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013183427 A1
- US 2020107568 A1
- WO 2005049839 A
- WO 2022012926 A1